# EUROPÄISCHE PATENTSCHRIFT

(11) **EP 1 015 597 B1**
(45) Veröffentlichungstag und Bekanntmachung des Hinweises auf die Patenterteilung: **13.08.2008**
(21) Anmeldenummer: 98954286.5
(22) Anmeldetag: 28.09.1998
(51) Int. Cl.: C12N 15/52, C12N 15/81, C12N 1/19, C12P 5/02, C12P 7/04, C12P 7/02, C12P 33/00

(54) **VERFAHREN ZUR HERSTELLUNG VON ERGOSTEROL UND DESSEN ZWISCHENPRODUKTEN MITTELS REKOMBINANTER HEFEN**
METHOD FOR PRODUCING ERGOSTEROL AND INTERMEDIATE PRODUCTS THEREOF BY MEANS OF RECOMBINANT YEASTS
PROCEDE DE PREPARATION D'ERGOSTEROL ET DE SES PRODUITS INTERMEDIAIRES A L'AIDE DE LEVURES RECOMBINEES

(30) Priorität: 30.09.1997 DE 19744212
(43) Veröffentlichungstag der Anmeldung: 05.07.2000
(73) Patentinhaber: OrganoBalance GmbH, 13355 Berlin (DE)
(72) Erfinder: WEBER, Alfred, D-14169 Berlin (DE); KLAGES, Uwe, D-13467 Berlin (DE); KENNECKE, Mario, D-14193 Berlin (DE); LANG, Christine, D-10625 Berlin (DE); STAHL, Ulf, D-13467 Berlin (DE); POLAKOWSKI, Thomas, D-13507 Berlin (DE)
(74) Vertreter: Jungblut, Bernhard Jakob
(86) Internationale Anmeldenummer: PCT/EP1998/006134
(87) Internationale Veröffentlichungsnummer: WO 1999/016886

(56) Entgegenhaltungen:
- EP-A- 0 313 465
- EP-A- 0 486 290
- V. ARIES AND B.H. KIRSOP: "Sterol Biosynthesis by strains of Saccharomyces cerevisiae in the presence and absence of dissolved oxygen" J. INST. BREWING, Bd. 84, Nr. 2, März 1978 - April 1978, Seiten 118-122, XP002094330 London, UK
- K. ALLEN ET AL.: "Effects of overproduction of the catalytic domain of 3-hydroxy-3-methylglutaryl coenzyme A reductase on squalene synthesis in Saccharomyces cerevisiae" APPLIED AND ENVIRONMENTAL MICROBIOL., Bd. 63, Nr. 9, September 1997, Seiten 3341-3344, XP002094331 AM. SOC. MICROBIOL.,WASHINGTON, DC, US
- N.D. LEES ET AL.: "Cloning of the late genes in the ergosterol Biosynthetic pathway of Saccharomyces cerevisiae" LIPIDS, Bd. 30, Nr. 3, März 1995, Seiten 221-226, XP002094332 AOCS Press,us
- M.E. BASSON ET AL.: "Saccharomyces cerevisiae contains two functional genes encoding 3-hydroxy-3-methylglutaryl-coenzyme A reductase" PROC. NATL. ACAD. SCI., Bd. 83, August 1986, Seiten 5563-5567, XP002094333 NATL. ACAD. SCI.,WASHINGTON,DC,US;
- S.M. JENNINGS ET AL.: "Molecular cloning and characterization of the yeast gene for squalen synthetase" PROC. NATL. ACAD. SCI., Bd. 88, Juli 1991, Seiten 6038-6042, XP002094334 NATL. ACAD. SCI.,WASHINGTON,DC,US;
- A. JANDROSITZ ET AL.: "The gene encoding squalene epoxidase from Saccharomyces cerevisiae: cloning and characterization" GENE, Bd. 107, 1991, Seiten 155-160, XP002094335 ELSEVIER SCIENCE PUBLISHERS,B.V.,AMSTERDAM,NL; in der Anmeldung erwähnt
- C. YU ET AL.: "Molecular cloning and characterization of two isoforms of Saccharomyces cerevisiae Acyl-CoA:Sterol Acyltransferase" J. BIOL. CHEM., Bd. 271, Nr. 39, 27. September 1996, Seiten 24157-24163, XP002094336 AM. SOC. BIOCHEM. MOL.BIOL.,INC.,BALTIMORE,US in der Anmeldung erwähnt
- POLAKOWSKI ET AL: "Overexpression of a cytosolic hydroxymethylglutaryl-CoA reductase leads to squalene accumulation in yeast" APPLIED MICROBIOLOGY AND BIOTECHNOLOGY, Bd. 49, 1998, Seiten 66-71, XP001167014
- POLAKOWSKI ET AL: "Inducing sterol esterification by engineering the sterol path[w]ay in yeast; Abstract 596, Yeast Genetics and Molecular Biology, College Park, Maryland, August 1998" 1998, Seite 1, XP001167015 Gefunden im Internet: URL:www.yeastgenome.org/yeast98/abshtml/59 6.html> [gefunden am 2004-11-22]
- VEEN ET AL: "Combined overexpression of genes of the ergosterol biosynthetic pathway leads to accumulation of sterols in Saccharomyces cerevisiae" FEMS YEAST RESEARCH, Bd. 4, 2003, Seiten 87-95,

### SEQUENCE LISTING

(1) GENERAL INFORMATION:
   (i) APPLICANT:
      (A) NAME: Schering AG
      (B) STREET: Müllerstrasse 178
      (C) CITY: Berlin
      (E) COUNTRY: Germany
      (F) POSTAL CODE (ZIP): D-13342
      (G) TELEPHONE: (030)-4681 2085
      (H) TELEFAX: (030)-4681 2058
   (ii) TITLE OF INVENTION: VERFAHREN ZUR HERSTELLUNG VON ERGOSTEROL UND DESSEN ZWISCHENPRODUKTEN MITTELS REKOMBINANTER HEFEN
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentin Release #1.0, Version #1.25 (EPO)
(2) INFORMATION FOR SEQ ID NO. 1:
   (i) SEQUENCE CHARACTERISTICS:
      (A) LENGTH: 25 bases
      (B) TYPE: nucleic acid
      (C) STRANDEDNESS: single
      (D) TOPOLOGY: linear
   (ii) HYPOTHETICAL: NO
   (iii) SEQUENCE DESCRIPTION: SEQ ID NO: 1:
      5'- ACTATGGACC AATTGGTGAA AACTG
(2) INFORMATION FOR SEQ ID NO. 2:
   (i) SEQUENCE CHARACTERISTICS:
      (A) LENGTH: 23 bases
      (B) TYPE: nucleic acid
      (C) STRANDEDNESS: single
      (D) TOPOLOGY: linear
   (ii) HYPOTHETICAL: NO
   (iii) SEQUENCE DESCRIPTION: SEQ ID NO. 2:
      5'- AGTCACATGG TGCTGTTGTG CTT
(2) INFORMATION FOR SEQ ID NO. 3:
   (i) SEQUENCE CHARACTERISTICS:
      (A) LENGTH: 25 bases
      (B) TYPE: nucleic acid
      (C) STRANDEDNESS: single
      (D) TOPOLOGY: linear
   (ii) HYPOTHETICAL: NO
   (iii) SEQUENCE DESCRIPTION: SEQ ID NO. 3:
      5'- GAATTCAACC ATGGACAAGA AGAAG
(2) INFORMATION FOR SEQ ID NO. 4:
   (i) SEQUENCE CHARACTERISTICS:
      (A) LENGTH: 24 bases
      (B) TYPE: nucleic acid
      (C) STRANDEDNESS: single
      (D) TOPOLOGY: linear
   (ii) HYPOTHETICAL: NO
   (iii) SEQUENCE DESCRIPTION: SEQ ID NO. 4:
      5'- AGAATTCCAC AGAACAGTTG CAGG

## Beschreibung

Die vorliegende Erfindung betrifft ein Verfahren zur Herstellung von Ergosterol und dessen Zwischenprodukten mittels rekombinanter Hefen und Plasmide zur Transformation von Hefen.

Ergosterol ist das Endprodukt der Sterol-Synthese in Hefen und Pilzen. Die wirtschaftliche Bedeutung dieser Verbindung liegt zum einen in der Gewinnung von Vitamin D₂ aus Ergosterol über UV-Bestrahlung, zum anderen in der Gewinnung von Steroidhormonen über Biotransformation, ausgehend von Ergosterol. Squalen wird als Synthesebaustein für die Synthese von Terpenen benutzt. In hydrierter Form findet es als Squalan Verwendung in Dermatologie und Kosmetik sowie in verschiedenen Derivaten als Inhaltsstoff von Haut- und Haarpflegemitteln. Ebenso von wirtschaftlicher Bedeutung sind die Zwischenprodukte des Ergosterol-Stoffwechselweges. Als wichtigste seien hier Farnesol, Geraniol und Squalen genannt. Weiterhin wirtschaftlich nutzbar sind Sterole, wie z.B. Zymosterol und Lanosterol, wobei Lanosterol Roh- und Synthesepivotal für die chemische Synthese von Saponinen und Steroidhormonen ist. Wegen seiner guten Hautpenetration und Spreadingeigenschaften dient Lanosterol als Emulsionshilfs- und Wirkstoff für Hautcremes.

Die Gene des Ergosterol-Stoffinrechsels in Hefe sind weitgehend bekannt und kloniert, so z.B. die HMG-CoA-Reduktase (*HMG1*) (Basson et al. (1988)),die Squalensynthetase (*ERG 9*) (Fegueur et al. (1991)), die Acyl-CoA:Sterol-Acyltransferase (*SAT1*) (Yu et al. (1996)) und die Squalenepoxidase (*ERG1*) (Jandrositz et al. (1991 )). Squalensynthetase katalysiert die Reaktion von Farnesylpyrophosphat über Presqualenpyrophosphat zu Squalen. Die Reaktionsmechanismen von Sterolacyltransferase sind nicht vollständig geklärt. Eine Überexpression der Gene dieser genannten Enzyme wurde bereits versucht, führte jedoch zu keiner nennenswerten Erhöhung der Ergosterol-Menge. Im Fall der *HMG1*-Überexpression wurde die Überproduktion von Squalen beschrieben, hierzu wurden zusätzlich Mutationen zur Unterbrechung des nach Squalen folgenden Weges eingeführt (EP-0 486 290). Die Überproduktion von Geraniol und Farnesol wurde ebenfalls beschrieben, hier erfolgte allerdings keine Überexpression von Genen des Ergosterol-Stoffwechsels, sondern eine Unterbrechung des Reaktionsweges in Richtung Geraniol- und Famesol-Bildung (EP-0313 465). Spezifische Inhibitoren der Ergosterolbiosynthese können ebenfalls zur Anhäufung größerer Mengen bestimmter Zwischenprodukte führen, z.B. Allylamine, die die Umwandlung von Squalen zu Squalenepoxid verhindern. Dadurch werden große Mengen (bis zum 600fachen des Normallevels) Squalen angehäuft (Jandrositz et al., (1991)). Aus der Literaturstelle N.D. Lees et al., Lipids, 30(3); 221-226 (1995) ist die Klonierung von fanen, die an der Biosynthese von Ergosterol beteiligt sind, bekannt. Zwar wurden durch die Verwendung von Inhibitoren eine große Anhäufung von z.B. Squalen erreicht, doch dürfte sich die Zugabe dieser Substanzen als nachteilig erweisen, da selbst geringe Mengen die gleiche Wirkung im Organismus entfalten, so daß eine Herstellung von Produkten der Ergosteralbiosynthese auf dem Weg der Überproduktion von Vorteil ist.

Siehe auch die folgenden Literaturstellen, die nach dem Prioritätstag veröffent-licht werden sind.

Polakowski et al: Overexpression of a cytosolic hydroxymethylglutaryl-CoA reductase leads to squalene accumulation in yeast, Applied Microbiology and Biotechnology, 49: 66-71 (1998).

Polakowski et al : Inducing sterol esterification by engineering the sterol path [w] ay in yeast, yeast genetics and Molecular Biology, College Park, Maryland Abstract 596, August 1998, www. yeastgenome.org/yeast 98 / abshtml/596.html

Aufgabe der vorliegenden Erfindung ist es, ein mikrobiologisches Verfahren zur Herstellung von Ergosterol und dessen Zwischenprodukten, die hierfür notwendigen Mikroorganismen wie Hefestämme, die erhöhte Mengen an Ergosterol bzw. hierfür notwendige Zwischenprodukte zu synthetisieren und die zur Transformation der Hefestämme notwendigen Plasmide bereitzustellen.

Es wurde nun gefunden, daß man die Menge an Ergosterol und dessen Zwischenprodukten steigern kann, wenn die Gene der *HMG1* (Basson et al., (1988)), *ERG9* (Fegueur et al., (1991)), Current Genetics 20: 365-372), *SAT1* (Yu et al., (1996)) und *ERG1* (Jandrositz et al. (1991)) in Mikroorganismen wie z.B. Hefen in veränderter Form eingebracht werden, wobei die Gene entweder einzeln auf einem Plasmid oder in Kombination auf einem oder mehreren Plasmiden lokalisiert sind und gleichzeitig oder nacheinander in den Wirt gebracht werden können.

Gegenstände der vorliegenden Erfindung sind in den Ansprüchen 1, 10, 11, 14 , 16, 17, 19 und 21 angegeben, Bevorzugte Ausführungs formen sind den Unteransprüchen entnehmbar.

Unter Zwischenprodukten sind Squalen, Farnesol, Geraniol, Lanosterol, Zymosterol, 4,4-Dimethylrymosteröl, 4-Methylzymosterol, Ergost-7-enol und Ergosta-5,7-dienol, insbesondere Sterole mit 5,7-Dienstruktur, zu verstehen.

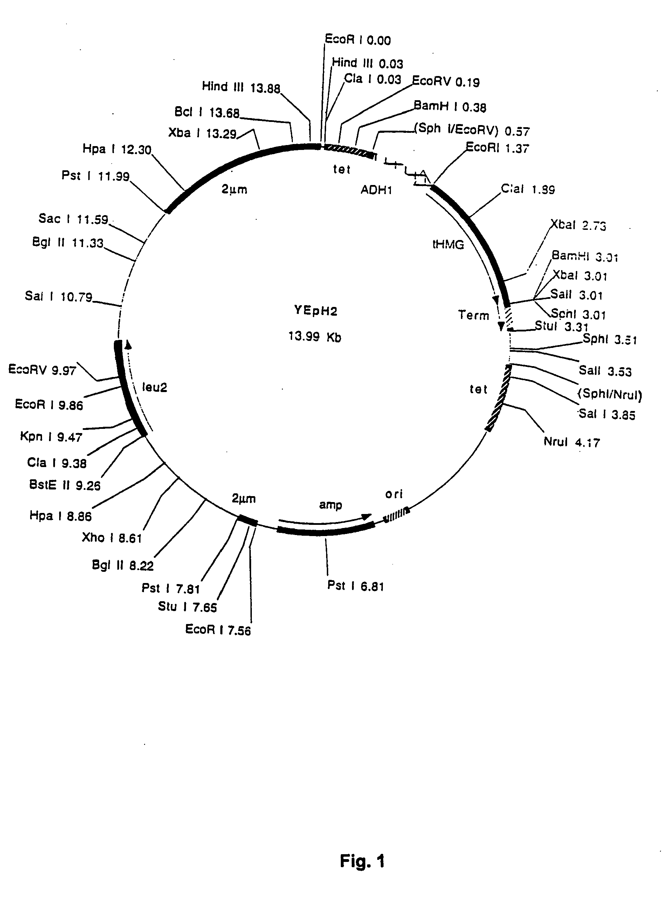

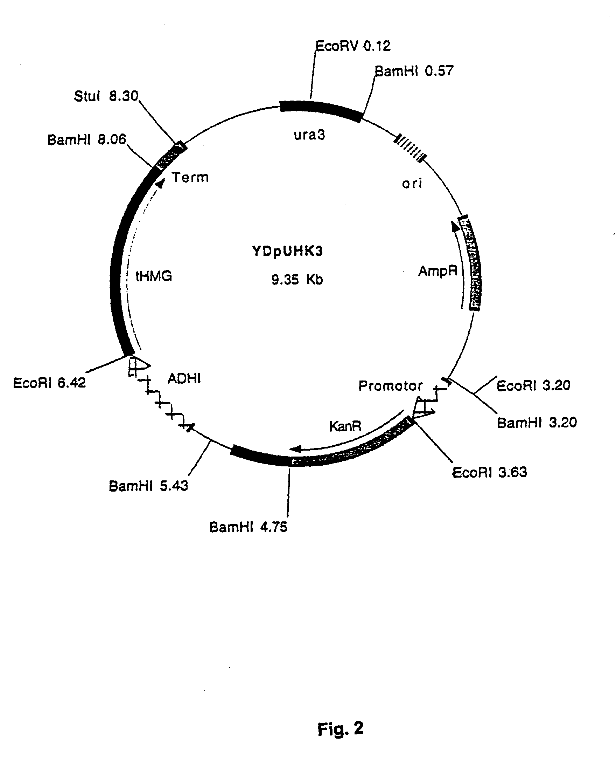

Die verwendeten Plasmide sind vorzugsweise das Plasmid YEpH2, das den mittleren *ADH*-Promotor, t-HMG (veränderte Variante des *HMG1*) und den *TRP-*Terminator (s. Fig. 1) enthält, das Plasmid YDpUHK3, das den mittleren *ADH-*Promotor, t-HMG (veränderte Variante des *HMG1*) und den *TRP*-Terminator, das Gen für die Kanamydn-Resistenz und das *ura3* Gen enthält (s. Fig. 2) und das Plasmid pADL-SAT1, das das *SAT1*-Gen und das *LEU2*-Gen aus YEp13 enthält.

Als Wirt für die Einführung der erfindungsgemäßen Plasmide kommen im Prinzip alle Mikroorganismen, insbesondere Hefen in Frage.

Weiterhin bevorzugt ist die kombinierte Transformation von Mikroorgansimen mit den Plasmiden pADL-SAT1 und YDpUHK3, insbesondere Hefen wie *S*. *cerevisiae* AH22.

Insgesamt gesehen wird der Fluß Im Ergosterol-Stcffwechselweg wie folgt beeinflußt
Der Fluß in Richtung Ergosterol wird maximiert, indem die Aktivität von mehreren Flaschenhals-Enzymen gleichzeitig verstärkt wird. Dabei spielen verschiedene Enzyme eine entscheidende Rolle, wobei die Kombination von Deregulation bzw. Überexpression den entscheidenden Durchbruch zur Ergosterolausbeutesteigerung bringt. Als Kombination werden die Enzyme bzw. deren Gene *HMG1* (Basson et al., (1988)), *ERG9* (Fegueur et al., (1991)), Acyl-CoA:Sterol-Acyltransferase *(SAT1)* (Yu et al. (1996)) und/ oder Squalenepoxidase (*ERG1*) (Jandrositz et al. (1991)) in einen Hefestamm in *veränderter* Form eingebracht, wobei das Einbringen der Gene mit einem oder mehrerer Plasmide erfolgt, wobei auf dem (den) Plasmid(en) die DNA-Sequenzen entweder einzeln oder in Kombination enthalten sind.
"*Verändert*" bedeutet im Falle des Gens *HMG1,* daß von dem entsprechenden Gen nur der katalytische Bereich ohne die membrangebundene Domäne exprimiert wird. Diese Veränderung wurde bereits beschrieben (EP-0486 290). Ziel der Veränderung von *HMG1* ist es, die Feed-back-Regulation durch Intermediate der Ergosterolbiosynthese zu verhindern. Sowohl *HMG1* ais auch die beiden anderen genannten Gene werden so auf gleiche Weise der transkriptionellen Regulation entzogen. Dazu wird der Promotor der Gene durch den "mittleren" *ADN1*-Promotor ersetzt. Dieses Promotorfragment des *ADH1-*Prornotors zeigt eine annähernd konstitutive Expression (Ruohonen et al., (1995)), so daß die transkriptionelle Regulation nicht mehr über Intermediate der Ergosterolbiosynthese abläuft.
Die bei der Überexpression entstehenden Produkte können in Biotransformationen bzw. anderen chemischen und therapeutischen Zwecken verwandt werden, z.B. die Gewinnung von Vitamin D₂ aus Ergosterol über UV-Bestrahlung, und die Gewinnung von Steroidhormonen über Biotransformation ausgehend von Ergosterol.

Bevorzugt ist eine veränderte Variante des Gens *HMG1,* in dem nur der katalytische Bereich ohne die membrangebundene Domäne exprimiert wird. Diese Veränderung ist beschrieben (EP-0486 290).

Die nachfolgenden Beispiele dienen der Erläuterung im Hinblick auf die Durchführung der für die Ausführungsbeispiele notwendigen Verfahren:

### 1. Restriktion

Die Restriktion der Plasmide ( 1 bis 10 µg) wurde in 30 µl Ansätzen durchgeführt. Dazu wurde die DNA in 24 µl H₂O aufgenommen, mit 3 µl des entsprechenden Puffers, 1 µl RSA (Rinderserumalbumin) und 2 µl Enzym versetzt. Die Enzymkonzentration betrug 1 Unit/µl oder 5 Units/µl je nach DNA-Menge. In einigen Fällen wurde dem Ansatz noch 1 µl RNase zugegeben, um die tRNA abzubauen. Der Restriktionsansatz wurde für zwei Stunden bei 37 °C inkubiert. Kontrolliert wurde die Restriktion mit einem Minigel.

### 2. Gelelektrophoresen

Die Gelelektrophoresen wurden in Minigel- oder Wide-Minigelapparaturen durchgeführt. Die Minigele (ca. 20 ml, 8 Taschen) und die Wide-Minigele (50 ml, 15 oder 30 Taschen) bestanden aus 1 %iger Agarose in TAE. Als Laufpuffer wurde 1 x TAE verwendet. Die Proben (10 µl) wurden mit 3 µl Stopperlösung versetzt und aufgetragen. Als Standard diente I-DNA geschnitten mit *Hin*dIII (Banden bei: 23,1 kb; 9,4 kb; 6,6 kb; 4,4 kb; 2,3 kb; 2,0 kb; 0,6 kb). Zur Auftrennung wurde eine Spannung von 80 V für 45 bis 60 min angelegt. Danach wurde das Gel in Ethidiumbromidlösung angefärbt und unter UV-Licht mit dem Video-Dokumentationssystem INTAS festgehalten oder mit einem Orange-Filter fotografiert.

### 3. Gelelution

Mittels Gelelution wurden die gewünschten Fragmente isoliert. Der Restriktionsansatz wurde auf mehrere Taschen eines Minigels aufgetragen und aufgetrennt. Nur λ-*Hin*dIII und eine "Opferspur" wurden in Ethidiumbromidlösung angefärbt, unter UV-Licht betrachtet und das gewünschte Fragment markiert. Dadurch wurde verhindert, daß die DNA der restlichen Taschen durch das Ethidiumbromid und das UV-Licht geschädigt wird. Durch Aneinanderlegen des gefärbten und ungefärbten Gelstücks konnte anhand der Markierung das gewünschte Fragment aus dem ungefärbten Gelstück herausgeschnitten werden. Das Agarosestück mit dem zu isolierenden Fragment wurde in einen Dialyseschlauch gegeben, mit wenig TAE-Puffer luftblasenfrei verschlossen und in die BioRad-Minigelapparatur gelegt. Der Laufpuffer bestand aus 1 x TAE und die Spannung betrug 100 V für 40 min. Danach wurde für 2 min die Strompolarität gewechselt, um am Dialyseschlauch klebende DNA wieder zu lösen. Der die DNA-Fragmente enthaltende Puffer des Dialyseschlauches wurde in Reaktionsgefäße überführt und damit eine EthanolFällung durchgeführt. Dazu wurde der DNA-Lösung 1/10 Volumen an 3 M Natriumacetat, tRNA (1 µl pro 50 µl Lösung) und dem 2,5 fachem Volumen an eiskaltem 96%igem Ethanol zugegeben. Der Ansatz wurde 30 min bei -20 °C inkubiert und dann bei 12000 rpm, 30 min, 4 °C abzentrifugiert. Das DNA-Pellet wurde getrocknet und in 10 bis 50 µl H₂O (je nach DNA-Menge) aufgenommen.

### 4. Klenow-Behandlung

Durch die Klenow-Behandlung werden überstehende Enden von DNA-Fragmenten aufgefüllt, so daß "blunt-ends" entstehen. Pro 1 µg DNA wurde folgender Ansatz zusammenpipettiert:

| | |
|---|---|
| DNA-Pellet | + 11 µl H₂O |
| | + 1,5 µl 10 x Klenow Puffer |
| | + 1 µl 0,1 M DTT |
| | + 1 µl Nucleotide (dNTP 2 mM) |
| | + 1 µl Klenow-Polymerase (1 Unit/µl) |

Die DNA sollte dabei aus einer Ethanolfällung stammen, um zu verhindern, daß Verunreinigungen die Klenow-Polymerase hemmen. Die Inkubation erfolgte für 30 min bei 37 °C, durch weitere 5 min bei 70 °C wurde die Reaktion abgestoppt. Die DNA wurde aus dem Ansatz durch eine Ethanolfällung gewonnen und in 10 µl H₂O aufgenommen.

### 5. Ligation

Die zu ligierenden DNA-Fragmente wurden vereinigt. Das Endvolumen von 13,1 µl enhielt ca. 0,5 µg DNA mit einem Vektor-Insert Verhältnis von 1:5. Die Probe wurde 45 Sekunden bei 70 °C inkubiert, auf Raumtemperatur abgekühlt (ca. 3 min) und dann 10 min auf Eis inkubiert. Danach wurden die Ligationspuffer zugegeben: 2,6 µl 500 mM TrisHCl pH 7,5 und 1,3 µl 100 mM MgCl₂ und weitere 10 min auf Eis inkubiert. Nach der Zugabe von 1 µl 500 mM DTT und 1 µl 10 mM ATP und nochmaligen 10 min auf Eis wurde 1 µl Ligase (1Unit/µl) zugegeben. Die ganze Behandlung sollte möglichst erschütterungsfrei erfolgen, um aneinanderliegende DNA-Enden nicht wieder zu trennen. Die Ligation erfolgte über Nacht bei 14 °C.

### 6. E. coli-Transformation

Kompetente *Escherichia coli* (*E. coli*) NM522 Zellen wurden mit der DNA des Ligationsansatzes transformiert. Als Positiv-Kontrolle lief ein Ansatz mit 50 ng des pScL3 Plasmids und als Null-Kontrolle ein Ansatz ohne DNA mit. Für jeden Transformationsansatz wurden 100 µl 8% PEG-Lösung, 10 µl DNA und 200 µl kompetente Zellen (*E*. *coli* NM522) in ein Tischzentrifugenröhrchen pipettiert. Die Ansätze wurden für 30 min in Eis gestellt und gelegentlich geschüttelt. Danach erfolgte der Hitzeschock: 1 min bei 42 °C. Für die Regeneration wurde den Zellen 1 ml LB-Medium zugegeben und für 90 min bei 37 °C auf einem Schüttler inkubiert. Je 100 µl der unverdünnten Ansätze, einer 1:10 Verdünnung und einer 1:100 Verdünnung wurden auf LB + Ampicillin-Platten ausplattiert und über Nacht bei
37 °C bebrütet.

### 7. Plasmid-Isolation aus E. coli (Minipräp)

*E*. *coli* -Kolonien wurden über Nacht in 1,5 ml LB + Ampicillin-Medium in Tischzentrifugenröhrchen bei 37 °C und 120 rpm angezogen. Am nächsten Tag wurden die Zellen 5 min bei 5000 rpm und 4 °C abzentrifugiert und das Pellet in 50 µl TE-Puffer aufgenommen. Jeder Ansatz wurden mit 100 µl 0,2 N NaOH, 1 % SDS-Lösung versetzt, gemischt und für 5 min auf Eis gestellt (Lyse der Zellen). Danach wurden 400 µl Na-Acetat/NaCl-Lösung (230 µl H₂O, 130 µl 3 M Natriumacetat, 40 µl 5 M NaCl) zugegeben, der Ansatz gemischt und für weitere 15 min auf Eis gestellt (Proteinfällung). Nach 15 minütiger Zentrifugation bei 11000 rpm wurde der Überstand, der die Plasmid-DNA enthält, in ein Eppendorfgefäß überführt. War der Überstand nicht vollständig klar, wurde nochmal zentrifugiert. Der Überstand wurde mit 360 µl eisgekühltem Isopropanol versetzt und für 30 min bei -20 °C inkubiert (DNA-Fällung). Die DNA wurde abzentrifugiert (15 min, 12000 rpm, 4 °C), der Überstand verworfen, das Pellet in 100 µl eisgekühltem 96%igem Ethanol gewaschen, 15 min bei -20 °C inkubiert und erneut abzentrifugiert (15 min, 12000 rpm, 4°C). Das Pellet wurde im Speed Vac getrocknet und dann in 100 µl H₂O aufgenommen. Die Plasmid-DNA wurde durch Restriktionsanalyse charakterisiert. Dazu wurden 10 µl jedes Ansatzes restringiert und in einem Wide-Minigel gelelektrophoretisch aufgetrennt (siehe oben).

### 8. Plasmid-Aufarbeitung aus E. coli (Maxipräp)

Um größere Mengen an Plasmid-DNA zu isolieren, wurde die Maxipräp-Methode durchgeführt. Zwei Kolben mit 100 ml LB + Ampicillin-Medium wurden mit einer Kolonie bzw. mit 100 µl einer Gefrierkultur, die das zu isolierende Plasmid trägt, angeimpft und über Nacht bei 37 °C und 120 rpm bebrütet. Die Anzucht (200 ml) wurde am nächsten Tag in einen GSA-Becher überführt und bei 4000 rpm (2600 x g) 10 min zentrifugiert. Das Zellpellet wurde in 6 ml TE-Puffer aufgenommen. Zum Abdau der Zellwand wurden 1,2 ml Lysozymlösung (20 mg/ml TE-Puffer) zugegeben und 10 min bei Raumtemperatur inkubiert. Anschließend erfolgte die Lyse der Zellen mit 12 ml 0,2 N NaOH, 1 % SDS-Lösung und weiteren 5 min Inkubation bei Raumtemperatur. Die Proteine wurden durch die Zugabe von 9 ml gekühlter 3 M Natriumacetat-Lösung (pH 4,8) und einer 15 minütigen Inkubation auf Eis gefällt. Nach der Zentrifugation (GSA: 13000 rpm (27500 x g), 20 min, 4 °C) wurde der Überstand, der die DNA enthielt, in einen neuen GSA-Becher überführt und die DNA mit 15 ml eiskaltem Isopropanol und einer Inkubation von 30 min bei -20 °C gefällt. Das DNA-Pellet wurde in 5 ml eisgekühltem Ethanol gewaschen und an der Luft getrocknet (ca. 30 - 60 min). Danach wurde es in 1 ml H₂O aufgenommen. Es fand eine Überprüfung des Plasmids durch Restriktionsanalyse statt. Die Konzentration wurde durch Auftragen von Verdünnungen auf einem Minigel bestimmt. Zur Verringerung des Salzgehaltes erfolgte eine 30 - 60 minütige Mikrodialyse (Porengröße 0,025-µm).

### 9. Hefe-Transformation

Für die Hefe-Transformation wurde eine Voranzucht des Stammes *Saccharomyces cerevisiae (S. cerevisiae*) AH22 angesetzt. Ein Kolben mit 20 ml YE-Medium wurde mit 100 µl der Gefrierkultur angeimpft und über Nacht bei 28 °C und 120 rpm bebrütet. Die Hauptanzucht erfolgte unter gleichen Bedingungen in Kolben mit 100 ml YE-Medium, die mit 10 µl, 20 µl oder 50 µl der Voranzucht angeimpft wurden.

### 9.1 Erstellen kompetenter Zellen

Am nächsten Tag wurden die Kolben mittels Thomakammer ausgezählt und es wurde mit dem Kolben, der eine Zellzahl von 3 - 5 × 10⁷ Zellen/ml besaß weitergearbeitet. Die Zellen wurden durch Zentrifugation (GSA: 5000 rpm (4000 x g), 10 min) geerntet. Das Zellpellet wurde in 10 ml TE-Puffer aufgenommen und auf zwei Tischzentrifugenröhrchen aufgeteilt (je 5 ml). Die Zellen wurden 3 min bei 6000 rpm abzentrifugiert und noch zweimal mit je 5 ml TE-Puffer gewaschen. Anschließend wurde das Zellpellet in 330 µl Lithiumacetat-Puffer pro 10⁹ Zellen aufgenommen, in einen sterilen 50 ml Erlenmeyerkolben überführt und eine Stunde bei 28 °C geschüttelt. Dadurch waren die Zellen kompetent für die Transformation.

### 9.2 Transformation

Für jeden Transformationsansatz wurden 15 µl Heringssperma DNA (10 mg/ml), 10 µl zu transformierende DNA (ca. 0,5 µg) und 330 µl kompetente Zellen in ein Tischzentrifugenröhrchen pipettiert und 30 min bei 28 °C (ohne Schütteln!) inkubiert. Danach wurden 700 µl 50% PEG 6000 zugegeben und für eine weitere Stunde bei 28 °C, ohne Schütteln, inkubiert. Es folgte ein Hitzeschock von 5 min bei 42 °C.
100 µl der Suspension wurden auf Selektionsmedium (YNB, Difco) ausplattiert, um auf Leucinprototrophie zu selektionieren. Im Falle der Selektion auf G418 Resistenz wird nach dem Hitzeschock eine Regeneration der Zellen durchgeführt (s. unter 9.3 Regenerationsphase)

### 9.3 Regenerationsphase

Da der Selektionsmarker die Resistenz gegen G418 ist, brauchten die Zellen Zeit für die Expression des Resistenz-Gens. Die Transformationsansätze wurden mit 4 ml YE-Medium versetzt und über Nacht bei 28 °C auf dem Schüttler (120 rpm) bebrütet. Am nächsten Tag wurden die Zellen abzentrifugiert (6000 rpm, 3 min) in 1 ml YE-Medium aufgenommen und davon 100 µl bzw. 200 µl auf YE + G418-Platten ausplattiert. Die Platten wurden mehrere Tage bei 28 °C bebrütet.

### 10. Reaktionsbedingungen für die PCR

Die Reaktionsbedingungen für die Polymerase Chain Reaction müssen für den Einzelfall optimiert werden und sind nicht uneingeschränkt für jeden Ansatz gültig. So kann unter anderem die eingesetzte Menge an DNA, die Salzkonzentrationen und die Schmelztemperatur variiert werden. Für unsere Problemstellung erwies es sich als günstig, in einem Eppendorfhütchen, das für den Einsatz im Thermocycler geeignet war, folgende Substanzen zu vereinigen: Zu 2 µl (∼0,1 U) Super Taq Polymerase wurden 5 µl Super Buffer, 8 µl dNTP's (je 0,625 µM), 5'-Primer, 3'-Primer und 0,2 µg Matritzen DNA, gelöst in soviel Wasser, daß sich ein Gesamtvolumen von 50 µl für den PCR Ansatz ergibt, zugegeben. Der Ansatz wurde kurz abzentrifugiert und mit einem Tropfen Öl überschichtet. Es wurden zwischen 37 und 40 Zyklen zur Amplifizierung gewählt.

Die nachfolgenden Ausführungsbeispiele beschreiben die Herstellung der erfindungsgemäßen Plasmide und Hefestämme sowie deren Verwendung, ohne jedoch die Erfindung auf diese Beispiele einzuschränken.

### Beispiel 1

### Expression der tHMG in S. cerevisiae AH22

Die DNA-Sequenz für *tHMG* (Basson et al., (1988)) wurde durch PCR aus genomischer DNA von *Saccharomyces cerevisiae* S288C (Mortimer und Johnston, (1986)) unter Anwendung von Standardmethoden amplifiziert. Die hierbei verwendeten Primer sind die DNA Oligomere tHMG-5' und tHMG-3' (s. Seq ID Nos. 1 und 2). Das erhaltene DNA-Fragment wurde nach einer Klenow-Behandlung in den Klonierungsvektor pUC19 (Yanisch-Perron et al., (1985)) eingebracht und ergab den Vektor pUC19-tHMG. Nach Plasmidisolation und Restriktion von pUC19-tHMG mit den Endonukleasen *Eco*RI und *Bam*HI wurde das gewonnene Fragment in den Hefeexpressionsvektor pPT2b (Lang und Looman, (1995)), der ebenfalls mit *Eco*RI und *Bam*HI behandelt wurde, eingebracht. Das entstandene Plasmid pPT2b-tHMG enthält den *ADH1-*Promotor (Bennetzen und Hall, (1982)) und den *TRP1*-Terminator (Tschumper und Carbon, (1980)), zwischen denen sich das *tHMG*-DNA-Fragment befindet. Aus dem Vektor pPT2b-tHMG wurde über die Endonucleasen *Eco*RV und *Nru*l ein DNA-Abschnitt isoliert, die den sogenannten mittleren *ADH1*-Promotor , die *tHMG* und dem *TRP1*-Terminator enthält. Dieser DNA-Abschnitt wurde in den Hefe-Vektor YEp13 (Fischhoff et al., (1984)), der mit der Endonuklease *Sph*I und einer DNA-Polymerase behandelt wurde, eingebracht. Der dadurch entstandene Vektor, der YEpH2 (Fig. 1), wurde mit den Endonukleasen *Eco*RV und *Nru*l behandelt. Es entstand so ein DNA-Fragment mit folgenden Bereichen: ein transkriptionsaktivie-render Bereich aus dem Tetracyclinresistenzgen (Sidhu und Bollon, (1990)), dem mittleren *ADH1-*Promotor, der *tHMG* und dem *TRP1*-Terminator (Expressionskassette). Dieses DNA-Fragment wurde in den Vektor YDpU (Berben et al., (1991)), der mit *Stu*l behandelt wurde, eingebracht. Der so entstandene Vektor YDpUH2/12 wurde mit der Endonuklease *Sma*l behandelt und mit einer DNA-Sequenz ligiert, die für eine Kanamycinresistenz kodiert (Webster und Dickson, (1983)). Das entstandene Konstrukt (YDpUHK3, Fig. 2) wurde mit *Eco*RV behandelt. Der Hefestamm *Saccharomyces cerevisiae* AH22 wurde mit diesem Konstrukt transformiert. Die Transformation der Hefe mit einem linearisierten Vektor, wie er in diesem Beispiel vorliegt, führt zu einer chromosomalen Integration des gesamten Vektors am URA3 Genlocus. Um die Bereiche aus dem integrierten Vektor zu eliminieren, die nicht zu der Expressionskassette gehören (*E.coli-*origin, *E.coli*-Ampicillin-Resistenzgen, TEF-Promotor und Kanamycin-Resistenzgen), wurden transformierte Hefen mittels FOA-Selektion (Boeke et al., (1987)) einem Selektionsdruck unterzogen, der Uracil-auxotrophe Hefen begünstigt. Der aus der Selektion hervorgegangene, Uracil-auxotrophe Stamm trägt die Bezeichnung AH22/tH3ura8 und besitzt die *tHMG1*-Expressionskassette als chromosomale Integration im *URA3*-Gen.

Der Hefestamm AH22/tH3ura8 und der Ausgangsstamm AH22 wurden 48 Stunden lang in YE bei 28°C und 160 rpm in Schikanekolben kultiviert.

Kultivierungsbedingungen: Die Vorkultur WMVIII wurde wie folgt angesetzt: 20 ml WMVIII + Histidin (20 µg/ml) + Uracil (20 µg/ml) wurden mit 100 µl Gefrierkultur angeimpft und 2 Tage bei 28°C und 120 rpm (reziprok) inkubiert. Aus der 20 ml Vorkultur wurden 100 ml WMVIII + Histidin (20 µg/ ml) + Uracil (20 µg/ ml) angeimpft. Für die Hauptkultur wurden 50 ml YE (in 250 ml Schikanekolben) mit 1 x 10⁹ Zellen beimpft. Die Kolben wurden bei 160 rpm auf einem Rundschüttler bei 28°C für 48 Stunden inkubiert. HMG-CoA-Reduktaseaktivitäten wurden (nach Qureshi et al., (1981)) bestimmt und ergaben folgenden Werte (s. Tabelle 1).

**Tabelle 1**

| | spezifische HMG-CoA-Reduktase-Aktivität* (U/mg Protein) |
|---|---|
| AH22 | 3,99 |
| AH22/tH3ura8 | 11,12 |

| | |
|---|---|
| *Ein Unit ist definiert als die Umsetzung von 1 nmol NADPH pro Minute in einem Milliliter Reaktionsgemisch. Die Messung erfolgte mit Gesamtproteinisolaten. | |

Die Sterole wurden extrahiert (Parks et al., (1985)) und über Gaschromatographie analysiert. Es ergaben sich folgende Werte (s. Tabelle 2).

**Tabelle 2**

| | Squalen (% w/w) | Ergosterol (% w/w) |
|---|---|---|
| AH22 | 0,01794 | 1,639 |
| AH22/tH3ura8 | 0,8361 | 1,7024 |

Die prozentualen Werte beziehen sich auf das Hefetrockengewicht.

### Beispiel 2

### Expression der SAT1 in S. cerevisiae AH22

Die Sequenz für die Acyl-CoA:Steroltransferase (*SAT1*; Yang et al., (1996)) wurde, wie oben beschrieben, durch PCR aus genomischer DNA von *Saccharomyces cerevisiae* S288C gewonnen. Die hierbei verwendeten Primer sind die DNA-Oligomere SAT1-5' und SAT1-3' (s. Seq ID Nos. 3 und 4). Das erhaltene DNA-Fragment wurde in den Klonierungsvektor pGEM-T (Mezei und Storts, (1994)) kloniert, was zum Vektor pGEM-SAT1 führte. Durch Behandlung von pGEM-SAT1 mit *Eco*RI erhielt man ein Fragment, das in den Hefeexpressionsvektor pADH1001, der ebenfalls mit *Eco*RI behandelt wurde, kloniert wurde. Der so entstandene Vektor pADH-SAT1 wurde mit der Endonuklease *Nru*I behandelt und mit einem Fragment aus YEp13, der das *LEU*2-Gen enthält, ligiert.

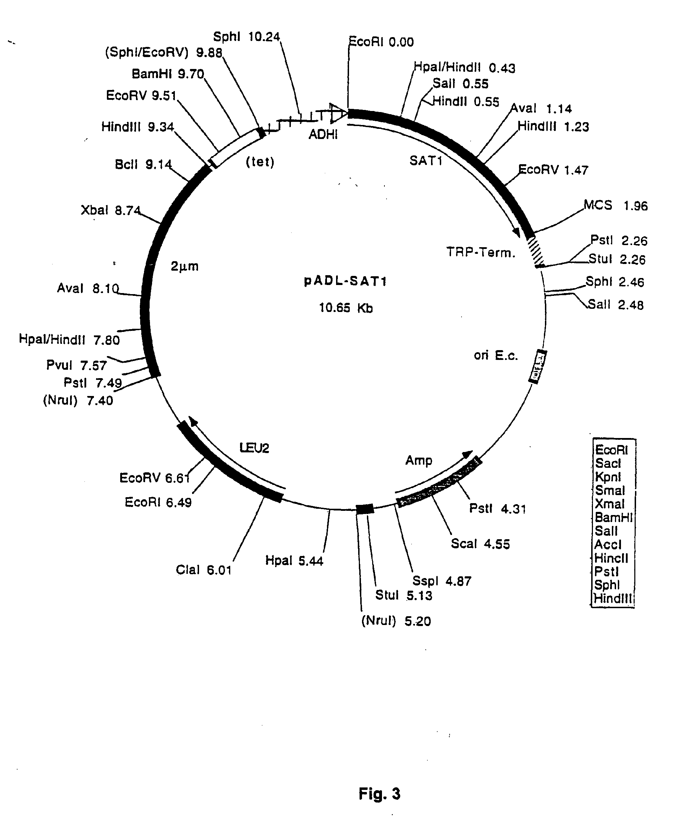

So entstand der Hefeexpressionsvektor pADL-SAT1 (Fig. 3), der in den Hefestamm AH22 eingebracht wurde. Der so gewonnene Stamm AH22/pADL-SAT1 wurde 7 Tage in WMVIII (Lang und Looman (1995)) Minimalmedium inkubiert. Kultivierungsbedingungen: (Zur Vorkultur s.o.) Hauptkultur: 50 ml WMVIII + Histidin (20µg/ml) + Uracil (20 µg/ml) Kulturen (in 250 ml Schikanekolben) wurden mit 1 x 10⁹ Zellen beimpft: Die Kolben wurden bei 160 rpm auf einem Rundschüttler bei 28°C für 7 Tage inkubiert. Die gebildeten Sterole wurden über Gaschromatographie analysiert (s. Tabelle 3).

**Tabelle 3**

| | Squalen (% w/w) | Ergosterol (% w/w) |
|---|---|---|
| AH22 | n.d. | 1,254 |
| AH22/ pADL-SAT1 | n.d. | 1,831 |

Die prozentualen Werte beziehen sich auf das Hefetrockengewicht. n.d.: nicht bestimmbar

### Beispiel 3

### Kombinierte Expression der verkürzten 3-Hydroxy-3-methylglutaryl-CoA-Reduktase (tHMG) und der Acyl-CoA:Sterol-Acyltransferase (SAT1)

### Beispiel 3.1

Der Hefestamm AH22/tH3ura8 wurde mit dem *SAT1*-Expressionsvektor pADL-SAT1 transformiert und ergab AH22/tH3ura8/pADL-SAT1. Dieser kombinierte Stamm wurde 7 Tage in WMVIII kultiviert. Die Sterole wurden extrahiert (s.o.) und über Gaschromatographie analysiert. Es ergaben sich folgende Werte (s. Tabelle 4).

**Tabelle 4**

| | Squalen (% w/w) | Ergosterol (% w/w) |
|---|---|---|
| AH22/tH3ura8 | 1,602 | 3,798 |
| AH22/tH3ura8/pADL-SAT1 | 1,049 | 5,540 |

Die prozentualen Werte beziehen sich auf das Hefetrockengewicht.

### Beispiel 3.2

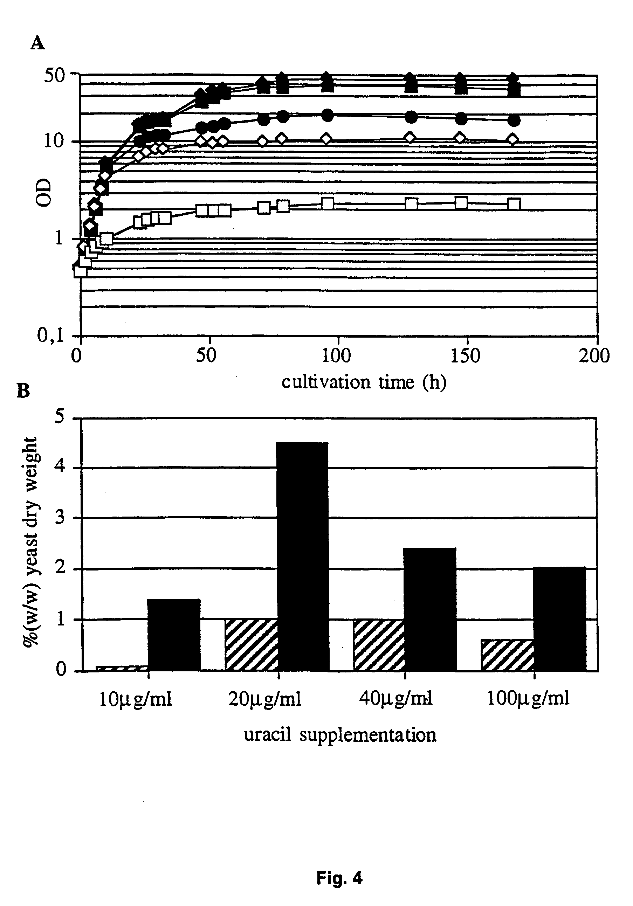

Hefekulturen wurden 7 Tage in WMVIII kultiviert, jedoch wurden verschiedene Mengen Uracil zu den Kulturen gegeben. Es wurden Konzentrationen von 10, 20, 40 und 100 µg/ml Uracil im Medium eingestellt. Die Ergosterol und die Squalenmengen sind bei einer Supplementation von 20 µg/ml Uracil maximal. Die Ergebnisse sind in der Fig. 4 dargestellt.

Es ist zu erkennen, daß die Ergosterol- und Squalenausbeute im Stamm AH22tH3ura8/pADL-SAT1 stark von der ins Kultivierungsmedium WMVIII zugegebenen Uracilmenge abhängig ist.

### Beispiel 3.3

Hefekulturen wurden 7 Tage in WMVIII kultiviert. Anschließend wurde die Gesamtheit der Sterole wie oben beschrieben bestimmt. Die freien Sterole werden aus mit Glasperlen aufgeschlossenen und mit n-Hexan extrahierten Hefen über GC bestimmt

Die Ergebnisse sind in der Tabelle 5 dargestellt.

Aus den Ergebnissen ist zu erkennen, daß das Enzym Sterol-Acyl Transferase (Sat1) mit hoher Effiktivität vornehmlich Sterole verestert, denen die 4,4-Diemthylgruppe fehlt. Damit kommt auch eine technische Anwendung für die Trennung von 4,4-Dimethylsterolen von den entsprechend demethylierten Formen in Betracht.

**Tabelle 5**

| Prozentualer Anteil freier Sterole. Jedes Sterol wurde als freies Sterol (ohne Verseifung) bestimmt und auf die Gesamtmenge dieses Sterols bezogen. In Klammem sind dazu die absoluten Gesamtsterolgehalte als Area/g Trockensubstanz angegeben. Lanosterol und 4,4-Dimethylzymosterol sind Sterole mit 4,4-Dimethylgruppe. | | |
|---|---|---|
| | | % freie Sterole |
| | Kontrolle | AH22tH3ura8/pADL-SAT1 |
| Lanosterol | 54 (0,99) | 59 (2,90) |
| 4,4-Dimethylzymosterol | 58 (0,77) | 84 (2,37) |
| 4-Methylzymosterol | 7 (2,43) | 10 (7,62) |
| Zymosterol | 10 (1,67) | 11 (5,85) |
| Ergost-7-enol, Ergosta-5,7-dienol | 24 (4,55) | 12 (9,00) |

### Beschreibung der Abbildungen

Fig. 1 zeigt das Plasmid YEpH2 mit den entsprechenden Schnittstellen.
Fig. 2 zeigt das Plasmid YDpUHK3 mit den entsprechenden Schnittstellen.
Fig. 3 zeigt das Plasmid pADL-SAT1 mit den entsprechenden Schnittstellen.
Fig. 4 zeigt das Wachstumsverhalten und Ergosterol- und Squalengehalte bei unterschiedlicher Uracilsupplementation. In der Abbildung bedeuten: OD = optische Dichte, cultivation time = Kultivierungszeit, yeast dry weight = Hefe-Trockengewicht, uracil supplementation = Uracilsupplementation.

### Literaturzitate

Basson, M.E., Thorsness, M., Finer-Moore, J., Stroud, R.M., Rine, J. (1988) Structural and functional conservation between yeast and human 3-hydroxy-3-methylgluataryl coenzyme A reductases, the rate-limiting enzyme of sterol biosynthesis. Mol. Cell. Biol. 8: 3793-3808.
Bennetzen, J. L., Hall, B. D. (1982) The primary structure of the Saccharomyces cerevisiae gene for alcohol dehydrogenase. J. Biol. Chem. 257: 3018-3025.
Berben, G., Dumont, J., Gilliquet, V., Bolle, P. A., Hilger, F. (1991) The YDp plasmids: a uniform set of vectors bearing versatile gene disruption cassettes for Saccharomyces cerevisiae. Yeast 7: 475-477.
Boeke, J. D., Trueheart, J., Natsoulis, G., Fink, G. (1987) 5-Fluorootic acid as a selective agent in yeast molecular genetics. Methods in Enzymology 154: 164-175.
Fegueur, M., Richard, L., Charles, A.D., Karst, F. (1991) Isolation ans primary structure of the ERG9 gene of Saccharomyces cerevisiae encoding squalene synthetase. Current Genetics 20: 365-372.
Fischhoff, D. A., Waterston, R. H., Olson, M. V. (1984) The yeast cloning vector YEp13 contains a tRNALeu3 gene that can mutate to an amber suppressor. Gene 27: 239-251.
Jandrositz, A., Turnowsky, F., Högenauer, G. (1991) The gene encoding squalen epoxidase from Saccharomyces cerevisiae: cloning and characterization. Gene 107: 155-160.
Mezei, L. M., Storts, D. R. (1994) in: PCR technology: current innovations, Griffin, H. G. and Griffin, A. M., eds. CRC Press, Boca Raton, 21.
Mortimer, R. K., Johnston, J. R. (1986) Genealogy of principal strains of the yeast genetic stock center. Genetics 113: 35-43.
Lang C., Looman A.C. (1995) Efficient expression and secretion of Aspergillus niger RH5344 polygalacturonase in Saccharomyces cerevisiae. Appl. Microbiol. Biotechnol. 44: 147-156.
Parks LW, Bottema CDK, Rodriguez RJ, Lewis TA (1985) Yeast sterols: yeast mutants as tools for the study of sterol metabolism. Meth. Enzymol. 111: 333-346.
Qureshi, N., Nimmannit, S., Porter, J. W. (1981) 3-Hydroxy-3-methylglutaryl-CoA reductase from Yeast. Meth. Enzymol. 71:455-461.
Ruohonen, L., Aalto, M.K., Keranen, S. (1995) Modifications to the ADH1 promoter of Saccharomyces cerevisiae for efficient production of heterologous proteins. Journal of Biotechnology 39: 193-203.
Siduh, R. S., Bollon, A. P. (1990) Bacterial plasmid pBR322 sequences serve as upstream activating sequences in Saccharomyces cerevisiae. Yeast 6: 221-229.
Tschumper, G., Carbon, J. (1980) Sequence of a yeast DNA fragment containing a chromosomal replicator and the TRP1 gene. Gene 10: 157-166.
Webster, T. D., Dickson, R. C. (1983) Direct selection of Saccharomyces cerevisiae resistant to the antiobiotic G418 following transformation with a DNA vector carrying the kanamycin-resistance gene of Tn903. Gene 26: 243-252.
Yang, H., Bard, M., Bruner, D. A., Gleeson, A., Deckelbaum, R. J., Aljinovic, G., Pohl, T. M., Rothstein, R., Sturley, S. L. (1996) Sterol esterification in yeast: a two-gene process. Science 272: 1353-1356.
Yanisch-Perron, C., Vieira, J., Messing, J. Gene 33 (1985) 103-119.
Yu, C., Rothblatt, J.A. Cloning and characterisation of the Saccharomyces cerevisiae acyl-CoA:sterol acyltrensferase (1996), The Journal of Biological Chemistry, 271: 24157-24163.

## Patentansprüche

1. Verfahren zur Herstellung von Ergosterol und dessen Zwischenprodukten,
**dadurch gekennzeichnet, dass** man
a-i) zunächst ein Plasmid konstruiert, auf dem folgende Gene inseriert sind:
i) das Gen der HMG-Co-A-Reduktase (t-HMG),
wobei t-HMG dahingehend verändert ist, dass der katalytische Bereich und nicht die membrangebundene Domäne exprimiert wird und der natürliche Promotor durch den mittleren ADH1- Promotor ersetzt wird,
ii) das Gen der Squalensynthetase (ERG9),
iii) das Gen der Acyl-CoA:Sterol-Acyltransferase (SAT1) und
iv) das Gen der Squalenepoxidase (ERG1),
oder
a-ii) zunächst ein Plasmid konstruiert, auf dem folgende Gene insertiert sind:
i) das Gen der HMG-Co-A-Reduktase (t-HMG) wobei t-HMG dahingehend verändert ist, dass der katalytische Bereich und nicht die membrangebundene Domäne exprimiert wird und der natürliche Promotor durch den mittleren ADH1- Promotor ersetzt wird,
und
ii) das Gen der Acyl-CoA: Sterol-Acyltransferase (SAT1),
a-iii) zunächst ein Plasmid konstruiert, auf dem folgende Gene insertiert sind:
i) das Gen der HMG-Co-A-Reduktase (t-HMG),
wobei t-HMG dahingehend verändert ist, dass der katalytische Bereich und nicht die membrangebundene Domäne exprimiert wird und der natürliche Promotor durch den mittleren ADH1- Promotor ersetzt wird,
und
iv) das Gen der Squalenepoxidase (ERG1)
b) zunächst Plasmide konstruiert, auf denen jeweils eines der unter a-i) genannten Gene insertiert ist,
wobei t-HMG dahingehend verändert ist, dass der katalytische Bereich und nicht die membrangebundene Domäne exprimiert wird und der natürliche Promotor durch den mittleren ADH1- Promotor ersetzt wird, und
c) mit den so hergestellten Plasmiden Mikroorganismen transformiert, wobei die Mikroorganismen mit einem Plasmid unter a-i) bis a-iii) transformiert werden oder mit mehreren Plasmiden unter b) gleichzeitig oder nacheinander transformiert werden,
d) mit den so hergestellten Mikroorganismen eine Fermentation zu Ergosterol durchführt,
e) nach erfolgter Fermentation das Ergosterol und dessen Zwischenprodukte aus den Zellen extrahiert und analysiert und schließlich
f) das so erhaltene Ergosterol und dessen Zwischenprodukte mittels Säulenchromatographie reinigt und isoliert.

2. Verfahren gemäß Anspruch 1, **dadurch gekennzeichnet, dass** auf dem Plasmid unter a-ii) zusätzlich das Gen der Squalenepoxidase (ERG1) und
auf dem Plasmid mit den folgenden insertierten Genen HMG-Co-A-Reduktase (t-HMG) und Squalensynthetase (ERG9) zusätzlich das Gen der Acyl-CoA: Sterol-Acyl-transferase insertiert ist.

3. Verfahren zur Herstellung von Ergosterol und dessen Zwischenprodukten, **dadurch gekennzeichnet, dass** man die in Anspruch 1 unter a-i) bis a-iiii) genannten Gene mit den Plasmiden zunächst jeweils unabhängig voneinander in Mikroorganismen gleicher Spezies einführt und mit diesen gemeinsam eine Fermentation zu Ergosterol durchführt und das so erhaltene Ergosterol aus den Zellen extrahiert, analysiert und mittels Säulenchromatographie reinigt und isoliert.

4. Verfahren gemäß den Ansprüchen 1 bis 3, **dadurch gekennzeichnet, dass** die Zwischenprodukte Squalen, Farnesol, Geraniol, Lanosterol, Zymosterol, 4.4-Dimethylzymosterol, 4-Methylzymosterol, Ergost-7-enol und Ergosta-5,7-dienol sind.

5. Verfahren gemäß den Ansprüchen 1 bis 3, **dadurch gekennzeichnet, dass** die Zwischenprodukte Sterole mit 5,7-Dienstruktur sind.

6. Verfahren gemäß den Ansprüchen 1 bis 3, **dadurch gekennzeichnet, dass** die Plasmide die Plasmide YEpH2, YDpUHK3 und pDL-SAT1 sind.

7. Verfahren gemäß den Ansprüchen 1 bis 3, **dadurch gekennzeichnet, dass** die Mikroorganismen Hefen sind.

8. Verfahren gemäß Anspruch 7, **dadurch gekennzeichnet, dass** es die Spezies *S. cerevisiae* ist.

9. Verfahren gemäß Anspruch 8, **dadurch gekennzeichnet, dass** es der Stamm *S. cerevisiae* AH22 ist.

10. Plasmid pADL-SAT1, bestehend aus dem SAT1-Gen und dem LEU2-Gen aus YEp13.

11. Verwendung der Plasmide gemäß Anspruch 10 zur Herstellung von Ergosterol.

12. Verwendung der Plasmide gemäß Anspruch 10 zur Herstellung der Ergosterol - Zwischenprodukte Squalen, Farnesol, Geraniol, Lanosterol, Zymosterol, 4,4-Dimethylzymosterol, 4-Methylzymosterol, Ergost-7-enol und Ergosta-5,7-dienol.

13. Verwendung der Plasmide gemäß Anspruch 10 zur Herstellung von Sterolen mit 5,7-Dienstruktur.

14. Expressionskassette, umfassend den mittleren ADH-Promotor, das t-HMG - Gen, den TRP - Terminator und das SAT1-Gen mit dem mittleren ADH - Promotor und dem TRP - Terminator.

15. Expressionskassetten, umfassend den mittleren *ADH*-Promotor, das *t-HMG*-Gen, den *TRP*-Terminator, das *SAT1*-Gen mit dem mittleren *ADH-*Promotor und dem *TRP*-Terminator und das ERG9-Gen mit dem mittleren *ADH*-Promotor und dem *TRP*-Terminator.

16. Kombination aus Expressionskassetten, wobei die Kombination aus
a) einer ersten Expressionskassette, auf der der *ADH*-Promotor, das *t-HMG*-Gen und der *TRP*-Terminator lokalisiert ist,
b) einer zweiten Expressionskassette, auf der der *ADH*-Promotor, das *SAT1*-Gen und der *TRP*-Terminator lokalisiert ist, und
c) einer dritten Expressionskassette, auf der der *ADH*-Promotor, das *ERG9*-Gen mit dem *TRP*-Terminator lokalisiert ist.

17. Verwendung der Expressionskassetten gemäß den Ansprüchen 14 bis 16, zur Transformation von Mikroorganismen, die bei der Fermentation zu Ergosterol eingesetzt werden.

18. Verwendung gemäß Anspruch 17, **dadurch gekennzeichnet, dass** der Mikroorganismus Hefe ist.

19. Mikroorganismen, enthaltend Expressionskassetten gemäß den Ansprüchen 14 bis 16.

20. Mikroorganismus gemäß Anspruch 19, **dadurch gekennzeichnet, dass** es Hefe ist.

21. Verwendung des Mikroorganismus gemäß den Ansprüchen 19 und 20, bei der Fermentation zu Ergosterol.

22. Verwendung des Mikroorganismus gemäß den Ansprüchen 19 und 20, bei der Fermentation zu Ergosterol-Zwischenprodukten.

## Claims

1. A method for producing ergosterol and intermediate products thereof, **characterized by** that
a-i) first, a plasmid is constructed, on which the following genes are inserted:
i) the gene of the HMG-Co-A reductase (t-HMG),
t-HMG being modified such that the catalytic section and not the membrane-bound domain is expressed, and the natural promoter is replaced by the middle ADH1 promoter,
ii) the gene of the squalene synthetase (ERG9),
iii)the gene of the acyl-CoA:sterol acyl transferase (SAT1),
iv) the gene of the squalene epoxidase (ERG1),
or
a-ii) first, a plasmid is constructed, on which the following genes are inserted:
i) the gene of the HMG-Co-A reductase (t-HMG),
t-HMG being modified such that the catalytic section and not the membrane-bound domain is expressed, and the natural promoter is replaced by the middle ADH1 promoter,
and
ii) the gene of the acyl-CoA:sterol acyl transferase (SAT1),
a-iii) first, a plasmid is constructed, on which the following genes are inserted:
i) the gene of the HMG-Co-A reductase (t-HMG),
t-HMG being modified such that the catalytic section and not the membrane-bound domain is expressed, and the natural promoter is replaced by the middle ADH1 promoter, and
iv) the gene of the squalene epoxidase (ERG1)
b) first, plasmids are constructed, on which one of the genes listed under a-i) is inserted,
t-HMG being modified such that the catalytic section and not the membrane-bound domain is expressed, and the natural promoter is replaced by the middle ADH1 promoter,
and
c) with the plasmids thus produced, microorganisms are transformed, the microorganisms being transformed with a plasmid under a-i) to a-iii) or with several plasmids under b), at the same time or one after the other,
d) with the microorganisms thus produced, fermentation to ergosterol is performed,
e) after completed fermentation, the ergosterol and the intermediate products thereof are extracted from the cells and analyzed, and finally
f) the ergosterol thus obtained and the intermediate products thereof are purified and isolated by means of column chromatography.

2. The method according to claim 1, **characterized by** that on the plasmid under a-ii) in addition the gene of the squalene epoxidase (ERG1) is inserted, and on the plasmid with the following inserted genes HMG-Co-A reductase (t-HMG) and squalene synthetase (ERG9) in addition the gene of the acyl-CoA:sterol acyl transferase is inserted.

3. A method for producing ergosterol and intermediate products thereof, **characterized by** that the genes with the plasmids listed in claim 1 under a-i) to a-iii) are first inserted independently from each other into microorganisms of the same species, and together with them fermentation to ergosterol is performed, and the thus obtained ergosterol is extracted from the cells, analyzed and purified and isolated by means of column chromatography.

4. The method according to claims 1 to 3, **characterized by** that the intermediate products are squalene, famesol, geraniol, lanosterol, zymosterol, 4,4-dimethyl zymosterol, 4-methyl zymosterol, ergost-7-sterol, and ergosta-5,7-dienol.

5. The method according to claims 1 to 3, **characterized by** that the intermediate products are sterols with 5,7-diene structure.

6. The method according to claims 1 to 3, **characterized by** that the plasmids are the plasmids YEpH2, YDdUHK3 and pDL-SAT1.

7. The method according to claims 1 to 3, **characterized by** that the microorganisms are yeasts.

8. The method according to claim 7, **characterized by** that it is the species *S. cerevisiae.*

9. The method according to claim 8, **characterized by** that it is the strain *S. cerevisiae* AH22.

10. The plasmid pADL-SAT1, consisting of the SAT1 gene and the LEU2 gene from YEp13.

11. The use of the plasmids according to claim 10 for producing ergosterol.

12. The use of the plasmids according to claim 10 for producing the ergosterol intermediate products squalene, famesol, geraniol, lanosterol, zymosterol, 4,4-dimethyl zymosterol, 4-methyl zymosterol, ergost-7-sterol, and ergosta-5,7-dienol.

13. The use of the plasmids according to claim 10 for producing sterols with 5,7-diene structure.

14. An expression cassette, comprising the middle ADH promoter, the t-HMG gene, the TRP terminator and the SAT1 gene with the middle ADH promoter and the TRP terminator.

15. Expression cassettes, comprising the middle ADH promoter, the t-HMG gene, the TRP terminator, the SAT1 gene with the middle ADH promoter and the TRP terminator and the ERG9 gene with the middle ADH promoter and the TRP terminator.

16. A combination of expression cassettes comprising
a) a first expression cassette, on which the ADH promoter, the t-HMG gene and the TRP terminator are localized,
b) a second expression cassette, on which the ADH promoter, the SAT1 gene and the TRP terminator are localized, and
c) a third expression cassette, on which the ADH promoter, the ERG9 gene with the TRP terminator are localized.

17. The use of expression cassettes according to claims 14 to 16 for the transformation of microorganisms that are used for the fermentation to ergosterol.

18. The use according to claim 17, **characterized by** that the microorganism is yeast.

19. Microorganisms, comprising expression cassettes according to claims 14 to 16.

20. The microorganism according to claim 19, **characterized by** that it is yeast.

21. The use of the microorganism according to claims 19 and 20 for the fermentation to ergosterol.

22. The use of the microorganism according to claims 19 and 20 for the fermentation to ergosterol intermediate products.

## Revendications

1. Procédé pour la production d'ergostérol et de produits intermédiaires ce celui-ci, **caractérisé en ce que**
a-i) d'abord, un plasmide est construit, sur lequel les gènes suivants sont insérés:
i) le gène de la HMG-Co-A réductase (t-HMG),
le t-HMG étant modifié de telle manière que la section catalytique et pas le domaine lié à la membrane soit exprimé, et le promoteur naturel soit remplacé par le promoteur ADH1 moyen,
ii) le gène de la squalène synthétase (ERG9),
iii) le gène de l'acyl-CoA:stérol acyltransférase (SAT1),
iv) le gène de la squalène-époxidase (ERG1),
ou
a-ii) d'abord, un plasmide est construit, sur lequel les gènes suivants sont insérés:
i) le gène de la HMG-Co-A réductase (t-HMG),
le t-HMG étant modifié de telle manière que la section catalytique et pas le domaine lié à la membrane soit exprimé, et le promoteur naturel soit remplacé par le promoteur ADH1 moyen,
et
ii) le gène de l'acyl-CoA:stérol acyltransférase (SAT1),
a-iii) d'abord, un plasmide est construit, sur lequel les gènes suivants sont insérés:
i) le gène de la HMG-Co-A réductase (t-HMG),
le t-HMG étant modifié de telle manière que la section catalytique et pas le domaine lié à la membrane soit exprimé, et le promoteur naturel soit remplacé par le promoteur ADH1 moyen,
et
iv) le gène de la squalène époxidase (ERG1)
b) d'abord, des plasmides sont construits, sur lesquels un des gènes mentionnés sous a-i) est inséré,
le t-HMG étant modifié de telle manière que la section catalytique et pas le domaine lié à la membrane soit exprimé, et le promoteur naturel soit remplacé par le promoteur ADH1 moyen, et
c) avec les plasmides tellement produits, des microorganismes sont transformés, les microorganismes étant transformés avec un plasmide sous a-i) à a-iii) ou avec plusieurs plasmides sous b), en même temps ou l'un après l'autre,
d) avec les microorganismes tellement produits, une fermentation à ergostérol est effectuée,
e) après la fermentation, l'ergostérol et les produits intermédiaires de celui-ci sont extraits des cellules et analysés, et enfin
f) l'ergostérol tellement obtenu et les produits intermédiaires de celui-ci sont purifiés et isolés au moyen de la chromatographie sur colonne.

2. Procédé selon la revendication 1, **caractérisé en ce que** sur le plasmide sous a-ii) additionnellement le gène de la squalène époxidase (ERG1) et
sur le plasmide avec les gènes insérés suivants HMG-Co-A réductase (t-HMG) et squalène synthétase (ERG9) additionnellement le gène de la l'acyl-CoA:stérol acyltransférase est inséré.

3. Procédé pour la production d'ergostérol et de produits intermédiaires de celui-ci, **caractérisé en ce que** les gènes avec les plasmides mentionnés dans la revendication 1 sous a-i) à a-iii) sont d'abord insérés indépendamment l'un de l'autre dans des microorganismes de la même espèce et communément avec ceux-ci une fermentation à ergostérol est effectuée, et l'ergostérol tellement obtenu est extrait des cellules, analysé et purifié et isolé au moyen de la chromatographie sur colonne.

4. Procédé selon les revendications 1 à 3, **caractérisé en ce que** les produits intermédiaires sont squalène, famésol, géraniol, lanostérol, zymostérol, 4,4-diméthyl-zymostérol, 4-méthyl-zymostérol, ergost-7-stérol, et ergosta-5,7-diénol.

5. Procédé selon les revendications 1 à 3, **caractérisé en ce que** les produits intermédiaires sont des stérols avec une structure de 5,7-diène.

6. Procédé selon les revendications 1 à 3, **caractérisé en ce que** les plasmides sont les plasmides YEpH2, YDdUHK3 et pDL-SAT1.

7. Procédé selon les revendications 1 à 3, **caractérisé en ce que** les microorganismes sont des levures.

8. Procédé selon la revendication 7, **caractérisé en ce qu'**elle est l'espèce *S. cerevisiae.*

9. Procédé selon la revendication 8, **caractérisé en ce qu'**elle est la souche *S. cerevisiae* AH22.

10. Plasmide pADL-SAT1, se composant du gène SAT1 et du gène LEU2 à partir de YEp13.

11. Utilisation des plasmides selon la revendication 10 pour la production d'ergostérol.

12. Utilisation des plasmides selon la revendication 10 pour la production des produits intermédiaires d'ergostérol squalène, famésol, géraniol, lanostérol, zymostérol, 4,4-diméthyl-zymostérol, 4-méthyl-zymostérol, ergost-7-stérol, et ergosta-5,7-diénol.

13. Utilisation des plasmides selon la revendication 10 pour la production de stérols avec une structure de 5,7-diène.

14. Cassette d'expression, comprenant le promoteur ADH moyen, le gène t-HMG, le terminateur TRP et le gène SAT1 avec le promoteur ADH moyen et le terminateur TRP.

15. Cassettes d'expression, comprenant le promoteur ADH moyen, le gène t-HMG, le terminateur TRP, le gène SAT1 avec le promoteur ADH moyen et le terminateur TRP et le gène ERG9 avec le promoteur ADH moyen et le terminateur TRP.

16. Combinaison de cassettes d'expression comprenant
a) une première cassette d'expression, sur laquelle le promoteur ADH, le gène t-HMG et le terminateur TRP sont localisés,
b) une deuxième cassette d'expression, sur laquelle le promoteur ADH, le gène SAT1 et le terminateur TRP sont localisés, et
c) une troisième cassette d'expression, sur laquelle le promoteur ADH, le gène ERG9 avec le terminateur TRP sont localisés.

17. Utilisation des cassettes d'expression selon les revendications 14 à 16 pour la transformation de microorganismes, qui sont utilisés pour la fermentation à ergostérol.

18. Utilisation selon la revendication 17, **caractérisée en ce que** le microorganisme est de la levure.

19. Microorganismes, comprenant des cassettes d'expression selon les revendications 14 à 16.

20. Microorganisme selon la revendication 19, **caractérisé en ce qu'**il est de la levure.

21. Utilisation du microorganisme selon les revendications 19 et 20 pour le fermentation à ergostérol.

22. Utilisation du microorganisme selon les revendications 19 et 20 pour le fermentation à des produits intermédiaires d'ergostérol.
